Europäisches Patentamt

European Patent Office

Office européen des brevets

(11) Veröffentlichungsnummer: **0 411 395 A2**

# EUROPÄISCHE PATENTANMELDUNG

(21) Anmeldenummer: 90113765.3

(51) Int. Cl.⁵: **A61K 31/40**

(22) Anmeldetag: **18.07.90**

(30) Priorität: **03.08.89 DE 3925759**

(43) Veröffentlichungstag der Anmeldung:
**06.02.91 Patentblatt 91/06**

(84) Benannte Vertragsstaaten:
**AT BE CH DE DK ES FR GB GR IT LI LU NL SE**

(71) Anmelder: **THERA-PATENT
VERWALTUNGS-GmbH
Griesberg 2
D-8031 Seefeld(DE)**

(72) Erfinder: **Die Erfinder haben auf ihre
Nennung verzichtet**

(74) Vertreter: **Behrens, Dieter, Dr.-Ing. et al
Wuesthoff & Wuesthoff Patent- und
Rechtsanwälte Schweigerstrasse 2e 2
D-8000 München 90(DE)**

(54) Verwendung von ACE-Inhibitoren für die Atheroskleroseprophylaxe.

(57) Die Erfindung betrifft die Verwendung der Inhibitoren des Angiotensin-Umwandlungsenzymus (ACE-Inhibitoren) zur Herstellung eines Arzneimittels für die Atheroskleroseprophylaxe bei Risikopatienten, etwa bei Risiken wie Nikotinabusus, Hypercholesterinämie, Diabetes und Hypertonie. Dazu werden die Inhibitoren, beispielweise Captopril in einer Menge von 0,5 bis 10 mg pro Tag oral mit üblichen galenischen Hilfs- und Trägerstoffen verabreicht.

EP 0 411 395 A2

## VERWENDUNG VON ACE-INHIBITOREN FÜR DIE ATHEROSKLEROSEPROPHYLAXE

Die Erfindung betrifft die Verwendung der Inhibitoren des Angiotensin-Umwandlungsenzymus (ACE-Inhibitoren) zur Herstellung eines Arzneimittels für die Atheroskleroseprophylaxe bei Risikopatienten.

An der Atherosklerose leidet die gesamte Menschheit. Sie ist eine Erkrankung der Gefäße, die mit dem Alter zunimmt. Es sterben heute die meisten Menschen an den Folgen: Herzinfarkt und Schlaganfall. Risiken wie Nikotinabusus, Hypercholesterinämie, Diabetes und Hypertonie können den Atheroskleroseprozeß beschleunigen, so daß eine signifikante Einengung der Gefäße und damit ein Herzinfarkt oder Schlaganfall früher auftritt. Nimmt man übergewichtige Stoffwechselgesunde ohne Nikotinabusus, Diabetes, Fettstoffwechselstörung oder Hypertonie, so kann man anhand der zunehmenden Pulswellengeschwindigkeit die jährlich zunehmende Starrheit der Gefäße messen.

Es ist bekannt, daß Inhibitoren des Angiotensin-Umwandlungsenzyms (Angiotensin-Converting-Enzyme oder ACE) eine starke blutdrucksenkende Wirkung beim essentiellen Hochdruck des Menschen zeigen. Derartige Inhibitoren wurden erstmals aus dem Gift der Schlange Bothrops jararaca isoliert und als Oligopeptide beschrieben; eine entsprechende Wirksamkeit wurde sowohl für das daraus erhaltene Pentapeptid mit der Bezeichnung $B.P.P._{5a}$ als auch für das Nonapeptid der Bezeichnung $B.P.P._{9a}$ oder SQ 20,881, letzteres mit der Aminosäurenfolge pyroGlu-Trp-Pro-Arg- Pro-Gln-Ile-Pro-Pro, nachgewiesen (siehe The Lancet 1973, 1972 und US-A-3 947 575). Ein solcher ACE-Inhibitor ist auch das Prolin-Derivat mit der chemischen Bezeichnung 1- (2S)-3-Mercapto-2-methylpropionyl -L-prolin, das unter der INN-Bezeichnung Captopril im Handel erhältlich ist.

Weitere ACE-Inhibitoren sind unter den Bezeichnungen Enalapril (N-(1-Ethoxycarbonyl-3-phenylpropyl)-L-alanyl-L-prolin), Lisinopril, Zofinopril und Ramipril erhältlich bzw. im arzneimittelrechtlichen Zulassungsverfahren (Zofinopril). Sie wurden bisher als Mittel zur Behandlung des erhöhten Blutdrucks und der Herzinsuffizienz verwendet.

Die Wirkung der ACE-Inhibitoren kann zum Einen mit einer Blockierung des Enzyms erklärt werden, welches im Organismus das Angiotensin I in das vasokonstriktorische und blutdruckwirksame Angiotensin II umwandelt. Im weiteren führt die Blockierung des Enzyms zu einer verminderten sympathischen Neurotransmission und letztlich zu einer Abnahme des Sympathikotonus, siehe auch New England Journal of Medicine, 1974, 817 sowie US-A-3 947 575.

Ferner ist bekannt, daß der Zusatz des ACE-Inhibitorpeptids $B.P.P._{9a}$ bzw. SQ 20881 zu bestimmten Extraktstoffen aus dem Bluteiweiß junger Kälber zu einer Verbesserung der mit diesen Extraktstoffen erzielten Wundheilung führt. Dies bedeutet, daß die Wirkung der Extraktstoffe durch den Zusatz der ACE-Inhibitoren verstärkt wird. Die Wirkung kann darauf zurückgeführt werden, daß durch die Kombination der Extraktstoffe mit den ACE-Inhibitoren der Stoffwechsel mangelhaft versorgter Gewebspartien durch eine verstärkte Glukoseaufnahme verbessert wird, wodurch speziell bei diabetischer Stoffwechsellage die Durchblutung verstärkt und, bei lokaler Anwendung, die Wundheilung beschleunigt wird, siehe DE-A-27 29 096 und DE-C-27 59 793.

Schließlich ist das Conversions-Enzym mit dem kininabbauenden Enzym Kininase II identisch, so daß unter Bedingungen der ACE- Inhibitoren mit einem verminderten Abbau und damit einer gesteigerten Konzentration von vasodilatorisch wirksamen Kininen zu rechnen ist.

Es wurde nun überraschend gefunden, daß ACE-Inhibitoren wie Captopril bei oraler Verabreichung in Mengen, die den Blutdruck nicht mehr wesentlich beeinflussen, an übergewichtigen Patienten, die keine Risiken für eine Gefäßsklerose hatten, die Progredienz der Atherosklerose anhand der Messung der Pulswellengeschwindigkeit signifikant zu vermindern in der Lage sind (Tab. 1).

Bei der Behandlung mit den erfindungsgemäß verwandten ACE-Inhibitoren handelt es sich um eine reine Propyhlaxe. Ein Medikament, das dies vermag, gibt es bisher nicht.

Für die erfindungsgemäße Verwendung werden die ACE-Inhibitoren z. B. Captopril in Mengen von 0,5 bis 15 mg/Tag, vorzugsweise 1 bis 10 mg/Tag oral verabreicht. Eine typische Dosierung für einen Erwachsenen ist beispielsweise 2 x 3 mg Captopril pro Tag, jedoch kann die Dosierung in Abhängigkeit vom Körpergewicht, Alter und physischem Zustand des Patienten in üblicher Weise variiert werden.

Der Wirkstoff für die erfindungsgemäße Verwendung ist für die orale Anwendung üblicherweise mit gängigen Hilfs- und Trägerstoffen formuliert. Dabei liegt die Einzeldosis vorzugsweise im Bereich von 0,5 bis 10 mg, insbesondere 1 bis 5 mg Captopril.

Bei der Behandlung von Bluthochdruck beträgt die Tagesdosis an Captopril beispielsweise 25 bis 150 mg pro Tag. Sie liegt also weit über der für die Atheroskleroseprophylaxe benötigten täglichen Menge.

Versuchsbericht

Es wurden 122 übergewichtige Personen ausgesucht, die im Mittel 25 % Übergewicht hatten. Diese Patienten gaben nach Aufklärung ihr Einverständnis, an dieser Studie über 3 Jahre teilzunehmen. Sie hatten bereits 5 bis 10 Versuche einer Gewichtsabnahme hinter sich, die alle erfolgreich verlaufen waren. Ein Diabetes mellitus war nach der Definition des WHO-Reports (Techn. Rep. Ser. 646 , 1 bis 80, 1980) bei diesen Patienten ausgeschlossen. Sie wurden in zwei gleich starke Gruppen eingeteilt, so daß nach Alter sowie Ausmaß und Dauer des Übergewichts kein Unterschied bestand. Die Patienten wurden in halbjährlichen Abständen ambulant kontrolliert. Wie zu Beginn wurden bei diesen Kontrollen folgende Blutwerte gemessen: Blutsenkung, Blutbild, Nüchternblutzucker, IGTT, Gewicht, Cholesterin und Triglyzeride, Serumglutamat-Oxalat-Transaminase (SGOT), Serumglutamat-Pyruvat-Transaminase (SGPT). Damit wurden den Stoffwechsel beeinträchtigende Krankheiten ausgeschlossen. Als Parameter der Atherosklerose wurde die dopplersonographisch bestimmte maximale Blutströmungsgeschwindigkeit in der Arteria dorsalis pedis verwendet. Alle Patienten hatten eine schmerzfreie Gehstrecke von über 200 m, die Arteria dorsalis pedis war gut tastbar. Oszillographisch fand sich kein Anhalt für eine Mediasklerose. Das Vorliegen einer Neuropathie wurde mit den üblichen klinischen Methoden ausgeschlossen.

Die erste Gruppe erhielt 2 x 3 mg Captopril täglich, die zweite Gruppe entsprechend Placebo. Die Patienten hatten die Auflage, keine den Stoffwechsel beeinträchtigenden Medikamente wie z.B. Diuretika, Betablocker oder dergleichen einzunehmen. In beiden Gruppen schieden über den Zeitraum von 3 Jahren 28 Patienten aus verschiedenen Gründen aus. Die Gruppen veränderten sich dadurch jedoch nicht so sehr, daß sie statistisch nicht mehr auswertbar gewesen wären. Unter Anwendung des Student's t-Tests (Snedecor, G.W., Cochran, W.E.: Statistikal Methods, 2. edition, Ames Iowa: Iowa State University Press, 1967) ergab sich nach 3 Jahren ein signifikant geringerer Abfall der maximalen Blutströmungsgeschwindigkeit in der Captoprilgruppe (p 0,05; Student's t-Test). Die Ergebnisse des Versuchs sind in der nachstehenden Tabelle 1 dargestellt.

Tabelle I

| VERLAUF DER MAXIMALEN BLUTSTRÖMUNGSGESCHWINDKEIT BEI RISIKOPATIENTEN[a] ÜBER 3 JAHRE | | | | | |
|---|---|---|---|---|---|
| UNTER CAPTOPRIL (C)[b] GEGENÜBER PLACEBO (P) | | | | | |
| ZEITRAUM | | 1985 - 1 | 1986 - 2 | 1987 - 2 | 1988 - 1 |
| ANZAHL | C | 63 | 60 | 54 | 48 |
| | P | 59 | 54 | 49 | 46 |
| ALTER (Jahre) | C | 55,3 ± 0,8 | 56,2 ± 0,9 | 57,4 ± 0,9 | 58,6 ± 1,0 |
| | P | 57,2 ± 1,1 | 58,4 ± 1,0 | 59,6 ± 1,3 | 60,3 ± 1,3 |
| GEWICHT (in % des Idealgew.)[c] | C | 126,0 ± 1,1 | 124,1 ± 1,6 | 125,2 ± 1,1 | 125,5 ± 1,6 |
| | P | 124,1 ± 1,3 | 126,0 ± 1,4 | 123,6 ± 2,1 | 124,1 ± 1,9 |
| MAXIMALE BLUTSTRÖMUNGSGESCHWINDIGK. (cm/s) | C | 13,6 ± 0,4 | 13,5 ± 0,3 | 13,4 ± 0,2 | 13,5 ± 0,2 |
| | P | 13,5 ± 0,5 | 13,1 ± 0,4 | 13,0 ± 0,3 | 12,9 ± 0,4[d] |

a Stoffwechselgesunde Übergewichtige; keine diabetogenen Medikamente
b 2 x 3 mg tgl.
c Idealgewicht: (Größe (cm) -100)-15%.
d Signifikanz bei $p < 0,05$; Student's t-Test.

**Ansprüche**

1. Verwendung der Inhibitoren des Angiotensin-Umwandlungsenzyms zur Herstellung eines Arzneimittels für die Atheroskleroseprophylaxe bei Risikopatienten.

2. Verwendung nach Anspruch 1, gekennzeichnet durch eine Menge von 0,5 bis 10 mg Wirkstoff je Dosierungseinheit.